# EUROPEAN PATENT APPLICATION

(11) **EP 2 088 184 A1**
(43) Date of publication of application: **12.08.2009**
(21) Application number: 09001637.9
(22) Date of filing: 05.02.2009
(51) Int. Cl.: C10G 7/12, C10G 11/00, B01D 3/00, B01D 3/32, B01D 3/42, C07C 4/00, F28D 21/00

(54) **Method and apparatus for capturing and using heat generated by the production of light olefins**

(30) Priority: 11.02.2008 US 69474
(71) Applicant: Stone & Webster Process Technology, Inc., Houston, TX 77077 (US)
(72) Inventor: Kurukchi, Sabah, Houston, TX 77057 (US); Keady, Ginger, Houston, TX 77094 (US); Coyle, Lois, Houston, TX 77025 (US)
(74) Representative: Schupfner, Georg

(57) **Abstract**

A system and method for capturing the heat generated by the various types of catalytic cracking of heavy hydrocarbons feedstocks for the production of a light olefins and using the captured heat in the operation of a C3 stripper or propane-propylene fractionator.

## Description

### BACKGROUND OF THE INVENTION

This invention relates to a system for capturing the heat generated by the catalytic cracking of a heavy hydrocarbon feedstock and using the captured low level heat to provide heat for the reboiler(s) of a C3 splitter or propane-propylene fractionator. The invention also relates to a method for separating C3 hydrocarbons, such as propane and propylene, from a light olefin feedstock and separating propane and propylene by capturing and using the heat generated in the catalytic cracking of a heavy hydrocarbon feedstock.

Light olefins such as ethylene, propylene and butylene are the basic building blocks for hundreds of petrochemical products used by consumers everyday. Light olefins are obtained by cracking a number of feedstocks, such as ethane, propane, butane and various refinery streams. Some examples for obtaining light olefins are described in United States Patent Nos. 4,832,919; 6,576,805 and 6,667,409. One method employed to prepare light olefins is steam cracking C2-C4 light paraffins or from refinery streams. More recently, methods for improving light olefin production by catalytic cracking heavy oils have been investigated. For example, Fluid Catalytic Cracking (FCC), Residue Fluid Catalytic Cracking (RFCC), Deep Catalytic Cracking (DCC), Catalytic Pyrolysis Process (CPP), Ultimate Catalytic Cracking (UCC) and High Severity (HS) FCC have been investigated and in some cases actually used to generate light olefin feed stocks from heavy oils.

In the typical catalytic cracking process, heavy feedstocks, such as vacuum gas oils and residues (hydrotreated and nonhydrotreated) are catalytically cracked in a reactor using 2-40 weight percent dispersion steam to produce a wide range of products. The resulting hot gases from the reactor are cooled, condensed and separated in the main fractionator producing a range of products, such as slurry oil, light cycle oil (LCO), heavy cycle oil (HCO) and overhead vapor stream. High level heat (greater than 150°C) from the catalytic cracking reactor can and has been recovered by steam generation in the main fractionator.

The overhead vapor released from the main fractionator consists of a raw gasoline product and lighter hydrocarbons together with steam, hydrogen and inert gas from the reactor. Traditionally, the overhead vapor from the main fractionator releases low level heat (less than 150°C) to the air and/ or air cooling water and is partially condensed. The resulting vapor/liquid stream, sometimes referred to as a wet gas, is separated and further processed. The wet gas is compressed and cooled in a multistage wet gas compressor. The low level heat from both the main fractionator overhead vapor stream and wet gas compressor has been unused and wasted in the past. In the prior art processes, there had been no successful attempts to integrate the low level heat produced in the main fractionator with any downstream processing. It would represent a notable advance in the state of the art if the cost of producing product propylene could be lowered while concomitantly increasing the overall thermal efficiency of the catalytic cracking process by making use of a heat source that previously not only was wasted, but required cost to reject to the environment.

In an effort to develop a more energy efficient and lower cost production of light olefins such as propane and propylene, it is an object of the present invention to capture or recycle at least part of the heat generated in the catalytic cracking reactor of the heavy hydrocarbons, and in particular the low level heat, and use that heat in the Operation of a C3 stripper or propane-propylene fractionator.

It is a further object of the present invention to use the hot overhead vapor from a main fractionator of a catalytic cracking system in a closed water recirculation system for operation of a C3 stripper or propane-propylene fractionator.

### SUMMARY OF THE INVENTION

These and other objects of the present invention are achieved by a system and method that captures the heat generated by various condensers and coolers in the production of light olefin feedstocks and uses the captured heat to operate the boilers and/or reboilers of a C3 stripper or propane-propylene fractionator.

The system of the present invention comprises at least one condenser and/ or cooler associated with the production of a light olefin feedstock, a closed water recirculation water system and a C3 stripper or propane-propylene fractionator. The closed water recirculation system further comprises at least one boiler or reboiler for heating the feedstock of the C3 stripper or propane-propylene fractionator. The at least one condenser or cooler is preferably a low level heat condenser or low level heat cooler associated with the production of a light olefin feedstock, preferably an overhead vapor stream from a main fractionator of a catalytic cracking unit.

The method of the present invention comprises the steps of heating water in a closed water recirculation system with heat produced by at least one condenser or cooler apparatus employed in the catalytic cracking of heavy hydrocarbons, pumping the heated water in the closed water recirculation system to a C3 stripper or propane-propylene fractionator, heating a feed to the C3 stripper or propane-propylene fractionator with the heated water in the closed water recirculation system and returning the water in the closed water recirculation system from the C3 stripper or propane-propylene fractionator to the at least one condenser or cooler for reheating. The at least one condenser or cooler is preferably a low level heat condenser or low level heat cooler associated with the catalytic cracking of heavy hydrocarbon, preferably an overhead vapor stream from a main fractionator of a catalytic cracking unit. It has been discovered that the heat emitted by the various low level heat condensers and coolers employed in the catalytic cracking of heavy hydrocarbons is ideal for generating the heat required to operate C3 strippers or propane-propylene fractionator.

The system and method of the present invention may also comprise an additional heat source for the water in the closed water recirculation system if the heat provided by the various condensers and coolers is found to be insufficient for the requirements of the C3 stripper or propane-propylene fractionator. The system and apparatus may also employ a water surge tank in the closed water recirculation system to regulate the water flow in the closed water recirculation system.

As used herein the term "C3 stripper" refers to devices that are designed to separate the three carbon or C3 components from a hydrocarbon. The term "propane¬propylene fractionator" refers to devices that are designed to separate propane and propylene. Some examples of various C3 strippers and fractionators can be found in United States Patent Nos. 4,753,667; 5,157,195; 6,218,589; and 6,576,805. In addition the terms "boiler" and "reboiler" are used interchangeably in this specification and Claims unless specifically noted.

In a preferred embodiment of the present invention, the light olefin feedstock is generated in an FCC, RFCC, DCC or CPP unit for the cracking of heavy oils. The heat for the closed water recirculation system is obtained from the main fractionator overhead vapor condensers(s) and main fractionator wet gas coolers.

The heat for the closed water recirculation system may also be obtained from coolers associated with intermediate streams from the main fractionator and gas plant, such as lean oil, LCO and Naphtha coolers and condensers or from equipment necessary to improve the emissions of the cracking unit, such as deoxo reactor effluent coolers. It is also within the scope of the present invention that any one or combination of the aforementioned low level heat sources can be used in the present invention.

In one embodiment of the present invention, the water in the closed water recirculation system is heated to about 80 to about 120°C, preferably about 85 to about 110°C, and most preferably about 90 to about 100°C by the condensers and/ or coolers. The heated water in the closed water recirculation system is cooled to about 50 to about 75°C, preferably to about 55 to about 70°C and most preferably about 60 to about 65°C, as it passes through the boiler(s) and or reboiler(s) employed by the C3 splitter or propane-propylene fractionator and before returning to the condenser or cooler heat source.

In another embodiment, the heated water in the closed water recirculation system is used to heat the reboiler of the C3 stripper or propane-propylene fractionator and also an intermediate zone of the C3 stripper or propane-propylene fractionator.

### BRIEF DESCRIPTION OF THE DRAWINGS

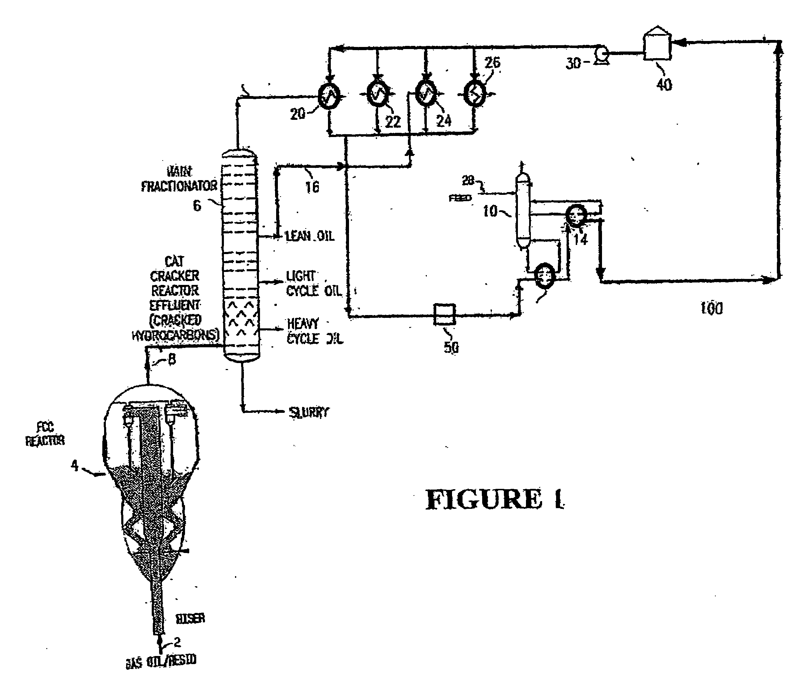

Figure 1 is simplified flow diagram of one embodiment of the present invention that shows a catalytic cracking unit 4, main fractionator 6 and closed water recirculation system 100.
Figure 1 is presented for illustration of the invention and does not include all the details of the invention such as pumps, instrumentation, compressors and similar hardware that are not essential to an understanding of the invention. Such miscellaneous equipment is well within the purview of one skilled in the art.

### DETAILED DESCRIPTION OF THE INVENTION

The present invention will be further described by reference to Figure 1. This description is not intended to limit the present invention or claims. It should be understood that the present invention can be applied to any type of catalytic cracking process for the catalytic cracking of heavy hydrocarbon feedstocks that employs condensers and coolers such as natural gas or other refinery streams. The present invention is also preferably employed in FCC, RFCC, DCC, CPP, UCC and HS FCC units. The application of the present invention to various catalytic cracking systems and feedstocks will be within the purview of one skilled in the art based upon the following detailed description.

As shown in Figure 1, a hydrocarbon feedstock, such as, but not limited to, a gas oil or resid, enters an FCC unit 4 via line 2 and is catalytically cracked using cracking conditions known to those skilled in the art, such as those described in United States Patent No. 6,576,805 which is incorporated by reference. The resulting hot cracked gases from the FCC unit 4 are sent via line 8 to a fractionator 6, sometimes referred to as a main fractionator, and are cooled, condensed and separated to produce a range of products such as slurry oil, HCO, LCO, lean oil and an overhead vapor stream 18. The overhead vapor 18 of the main fractionator 6 comprises a variety of materials, such as raw gasoline product, lighter hydrocarbons, steam and inert gases.

The resulting vapor/liquid stream is further processed for separation into products and/ or feedstocks for the petrochemical industry. In one step of the further process, the cracked gas stream is cooled and partially condensed in a condenser 20.

The partially condensed stream is then compressed and cooled in a multistage gas compressor system, commonly referred to as a wet gas compressor system, to remove water and condensable heavier hydrocarbons from the stream. The multistage wet gas compressor system can comprise a number of condensers and coolers in order to remove water and other condensables from the process stream, as is known to those skilled in the art. The arrangement of the various condensers and coolers will vary depending upon the composition of the Feed into the fractionator 6. Figure 1 only shows one cooler 22 associated with the multi stage wet gas compression by way of example. The vapor exiting the wet gas compression scheme may then be further processed, as is known to those skilled in the art, for separation of the stream into its component parts for use in the petrochemical industry, such as methane, ethane, C3 feeds and C4 feeds. For example, in a downstream depropanizer (not shown), the C3 hydrocarbons (propane and propylene) are separated as an overhead vapor stream 28 from C4 and heavier components. The C3 hydrocarbons from the depropanizer in line 28 are then typically separated in a C3 splitter 10, discussed in more detail herein below.

In addition to overhead vapor 18, the main fractionator 6 may also produce a lean oil component than can be used in later stages of the FCC processes, such as an absorber in an ethane and methane separator. The lean oil stream 16 from the main fractionator 6 can be cooled in cooler 24 prior to its use as an absorber. The lean oil cooler 24 is also a potential low level heat source for the closed water recirculation system of the present invention.

It is also well known that catalytic cracking and any hydrocarbon processing facility will require numerous scrubbers and reactors to reduce the environmentally harmful emissions of the facility. It is within the scope of the present invention to capture and reuse the heat, preferably the low level heat, generated by the scrubbers and reactors. By way of example, a deoxo reactor effluent cooler 26 can be used to heat the water in the closed water recirculation system of the present invention.

The water in the closed water recirculation system 100 of the present invention travels through one or more of the heat exchanger(s) 20-26 (it is contemplated by the present invention that there may be more or less than the four shown in Figure 1) associated with the condensers and coolers and is heated to about 80 to about 120°C, preferably about 85 to about 110°C, and most preferably about 90 to about 100°C. The heated water in the closed water recirculation system 100 then travels to a boiler or reboiler 12 associated with the C3 splitter or propane-propylene fractionator 10. The heated water in the closed water recirculation system may also optionally -travel through a second boiler or reboiler 14 that heats an intermediate zone of the C3 splitter or propane-propylene fractionator 10. Although a C3 stripper may be used in the present invention, it is preferred that propane-propylene fractionator, sometimes also referred to as a propane-propylene splitter or C3 splitter, be employed.

The water in the closed water recirculation system 100 is cooled in the boiler or reboiler 12 and optionally 14 to about 50 to about 75°C, preferably to about 55 to about 70°C and most preferably about 60 to about 65°C before returning to the condenser/ cooler heat source.

The closed water recirculation system 100 may also employ pumps 30 and/ or surge tanks 40 to maintain appropriate flow and pressure in the system.

In operation, it is believed that vapor 18 entering the overhead vapor condenser 20 will be about 150°C to about 110°C and the exit temperature will be about 75°C to about 85°C. The temperature of the wet gas entering the first stage of the multistage wet gas compressor 22 will be about 105°C to about 115°C and the temperature of the stream exiting the multistage wet gas compressor will be about 70° C. The temperature of the stream entering the lean oil cooler 24 will be about 130°C, while the exit temperature will be about 70°C. Finally, the temperature of the feed entering the deoxo reactor cooler 26 is believed to be about 140° C and the exit temperature is about 70°C.

In a preferred embodiment of the present invention, the water in the closed water recirculation system 100 entering the various condensers and coolers 20-26 will be about 60°C to about 65°C.

This water in the closed water recirculation system 100 will be heated to about 90°C to about 110°C in the various condensers and coolers 20-26. The temperature of the water in the closed water recirculation system 100 exiting the various condensers and coolers 20-26 will naturally depend upon the temperature of the feed entering the condenser or cooler and the contact time with the water in the recirculation system 100. For example, it is believed that the temperature of the water in the closed water recirculation system exiting the overhead vapor condenser 20 and multistage wet gas compressor(s) 22 will be approximately 90°C and the temperature of the water in the closed water recirculation system 100 existing the lean oil cooler 24 and deoxo reactor effluent cooler 26 will be about 110°C. The heated water in the closed water recirculation system 100 from all the various condensers and coolers 20-26 will be combined to provide water to the C3 stripper or propane-propylene fractionator 10 at a preferred temperature of about 90 to about 95°C. It is believed that this water supply temperature will allow the C3 feedstock to be heated to about 65°C which in turn will allow the Separation of the various C3 components and preferably the separation of the propane and propylene.

As discussed previously, the water from the C3 splitter or propane-propylene fractionator boiler or reboiler 12 may be pumped to a second or intermediate stage reboiler 14. The second or intermediate reboiler 14 will heat an intermediate stage component of the C3 stripper or propane-propylene fractionator to about 55°C. The water from the boiler 12 and/ or optional intermediate boiler 14 may be pumped to a hot water surge tank 40 via pump 30 prior to reheating by the various condensers and coolers 20-26. The surge tank 40 and pump(s) 30 will be used to regulate the pressure and flow of the closed water recirculation system.

If necessary a supplemental heater 50, preferably heated with heat from a low pressure steam source, may be added to the closed water recirculation system of the present invention. The supplemental heater will insure that the heating requirements of the boiler 12 and/ or intermediate boiler 14 are meet.

While certain preferred and alternative embodiments of the invention have been set forth for purposes of disclosing the invention, modifications to the disclosed embodiments may occur to those who are skilled in the art.

Accordingly, the appended claims are intended to cover all embodiments of the invention and modifications thereof which do not depart from the spirit and scope of the invention.

## Claims

1. A system for capturing and using heat generated during the catalytic cracking of heavy hydrocarbon feedstocks comprising:
- at least one condenser or cooler associated with the catalytic cracking unit;
- a closed water recirculation system comprising water that is indirectly heated by the at least one condenser or cooler and provides indirect heat to at least one reboiler for a C3 stripper or propane-propylene fractionator.

2. The system according to claim 1 wherein the light olefin feedstock is produced by a catalytic cracking unit.

3. The system according to claim 1 or 2 wherein the at least one condenser or cooler is a condenser or cooler for overhead vapors of a main fractionator of the catalytic cracking unit.

4. The system accrdong to claim 1 or 2 wherein the at least one condenser or cooler is a condenser or cooler for an intermediate sidestream from a main fractionator of the catalytic cracking unit.

5. The system according to claim 1 or 2 wherein the at least one condenser or cooler is a condenser or cooler for an environmental scrubber or reactor for the catalytic cracking unit.

6. The system according to claim 1 or 2 wherein the at least one condenser or cooler is a low level heat condenser or cooler.

7. The system according to at least one of the preceding claims wherein the closed water recirculation system comprises a bottoms reboiler for the propane-propylene fractionator.

8. The system according to claim 1 or 7 wherein the water supplied as an indirect heat source for the bottoms reboiler to the propane-propylene fractionator is at a temperature ranging from about 80 to about 120°C, preferably about 85 to about 110°C, and most preferably about 90 to about 100°C.

9. The system according to claim 1, 7 or 8 wherein the water exiting the bottoms reboiler for the propane-propylene fractionator is at a temperature ranging from about 50 to about 75°C, preferably to about 55 to about 70°C and most preferably about 60 to about 65°C.

10. A method for capturing and using heat generated during the production of a light olefins comprising the following steps:
- heating water in a closed water recirculation system with heat produced by at least one condenser and/ or cooler apparatus employed in the production of a light olefin feedstock;
- pumping the heated water in the closed water recirculation system to a reboiler for a downstream C3 stripper or propane-propylene fractionator;
- said heated water providing indirect heating for the reboiler of the C3 stripper or propane-propylene fractionator; and returning the cooled water exiting the reboiler of the C3 stripper or propane-propylene fractionator in the closed water recirculation system from the C3 stripper or propane-propylene fractionator to the at least one condenser and/ or cooler for reheating.

11. The method according to claim 10 wherein the light olefins are produced by a catalytic cracking unit.

12. The method according to claim 10 or 11 wherein the at least one condenser or cooler is
a) a condenser or cooler for overhead vapors of a main fractionator of the catalytic cracking unit; or
b) a condenser or cooler for an intermediate side stream of a main fractionator of the catalytic cracking unit; or
c) is a condenser or cooler for an environmental scrubber or reactor for treating the cracked gases from the catalytic cracking unit; or
d) a low level heat condenser or cooler.

13. The method according to at least one of claims 10 to 12 wherein the closed water recirculation system comprises a bottoms reboiler for a propane-propylene fractionator.

14. The method according to at least one of claims 10 to 13 wherein the water to the bottoms reboiler is at a temperature ranging from about 80 to about 120°C, preferably about 85 to about 110°C, and most preferably about 90 to about 100°C.

15. The method according to at least one of claims 10 to 14 wherein the cooled water exiting the bottoms reboiler is at a temperature ranging from about 50 to about 75°C, preferably to about 55 to about 70°C and most preferably about 60 to about 65°C.
